# EUROPEAN PATENT SPECIFICATION

(11) **EP 2 832 216 B1**
(45) Date of publication and mention of the grant of the patent: **27.03.2019**
(21) Application number: 12872692.4
(22) Date of filing: 30.03.2012
(51) Int. Cl.: A01K 29/00, A01K 67/00, A61D 17/00, A61D 99/00

(54) **NOTIFICATION METHOD, NOTIFICATION PROGRAM, AND NOTIFICATION DEVICE**
BENACHRICHTIGUNGSVERFAHREN, BENACHRICHTIGUNGSPROGRAMM UND BENACHRICHTIGUNGSVORRICHTUNG
PROCÉDÉ DE NOTIFICATION, PROGRAMME DE NOTIFICATION ET DISPOSITIF DE NOTIFICATION

(43) Date of publication of application: 04.02.2015
(73) Proprietor: FUJITSU LIMITED, Kawasaki-shi Kanagawa 211-8588 (JP)
(72) Inventor: UCHINO, Tetsuya, Fukuoka-shi Fukuoka 814-8589 (JP); MOTOSHIMA, Toshimi, Fukuoka-shi Fukuoka 814-8589 (JP); KANAMORI, Akihito, Fukuoka-shi Fukuoka 814-8589 (JP); INENAGA, Mitsuhisa, Fukuoka-shi Fukuoka 814-8589 (JP); WATANABE, Katsuyoshi, Kawasaki-shi Kanagawa 211-8588 (JP); YAMANO, Daiji, Kawasaki-shi Kanagawa 211-8588 (JP)
(74) Representative: Haseltine Lake LLP
(86) International application number: PCT/JP2012/058759
(87) International publication number: WO 2013/145328

(56) References cited:
- EP-A2- 2 428 113
- CN-A- 102 100 185
- JP-A- H1 156 147
- JP-A- 2008 092 878
- JP-A- 2008 148 569
- US-A- 6 099 482
- US-A1- 2008 147 458
- US-A1- 2010 198 023
- US-A1- 2011 125 065

## Description

### TECHNICAL FIELD

The present invention relates to a notifying method, a notifying program, and a notifying apparatus.

### BACKGROUND ART

Conventionally, when livestock animals such as cattle are raised on a pasture, a worker of a livestock farm periodically goes to a pasture area to visually observe and medically examine the livestock animals individually to check for disease. Related techniques includes a proposed technique in which start timing of cattle disease is detected by comparing a measurement result of the past step count and a measurement result of the current step count (see, e.g., Patent Document 1).

Patent Document 1: Japanese Laid-Open Patent Publication No. 2008-92878

### DISCLOSURE OF INVENTION

### PROBLEM TO BE SOLVED BY THE INVENTION

Nonetheless, the conventional technique has a problem in that the onset of a disease cannot be detected from a measurement result of the past step count in some cases. For example, if a measurement result of the step count of a livestock animal already having a disease is used as the measurement result of the past step count, the onset of the disease cannot be detected in some cases since a change becomes smaller even when a comparison is made between the step count after the onset and the current step count.

To solve the problems of the conventional technique described above, an object of the present invention is to provide a notifying method, a notifying program, and a notifying apparatus that can support the finding of a livestock animal having a disease.

United States patent application publication US2010198023A1 discloses a computerized system and method for livestock groups health monitoring comprising: a storage and computing unit storing at least one database; a plurality of data collecting units of different types, each type comprising at least one sensor, the data collecting unit types selected from the group consisting of acoustic sensors, vitality meters, ammonia sensors, visual sensors and scent sensors; first communication means for communicating operating commands from the storage and computing unit to each of the data collecting units and for communicating data from the data collecting units to the storage and computing unit; and second communication means for communicating between the storage and computing unit and a user device.

United States patent application publication US2008147458A1 discloses a breeding support system including at least one information terminal for processing breeding information associated with breeding work of at least one individual breeding object, the system comprising: individual information collecting means for collecting individual information indicating a time-variable change of an individual condition of the individual breeding object; specified breeding work requirement determination means for determining requirement for a specified breeding work from among all breeding work in accordance with a profile of the time-variable change of the individual condition; breeding guidance generation means for generating at least one breeding guidance announcing a requirement of the specified breeding work determined; and breeding guidance supply means for supplying the generated breeding guidance as the breeding information to the information terminal.

United States patent application publication US2011125065A1 discloses a method and device for detecting estrus in animals by sensing along time the motion of the animal and identifying when the sensed motion is not related to eating periods of the animal. Based on the sensed motion which is not related to eating periods the estrus in the animal is identified.

Chinese patent application publication CN102100185A discloses a cow estrus monitoring system. The system is characterised by comprising at least one acceleration detection unit arranged on a cow, wherein all the acceleration detection units are connected with a pace analysis unit so as to acquire and analyze cow pace information; the pace analysis unit is connected with a wireless communication transmitting unit; the wireless communication transmitting unit is connected with a wireless communication receiving unit in a wireless mode; and the wireless communication receiving unit is connected with an upper computer so as to store the cow pace information and analyze the current state of the cow by combining a preset program. The monitoring system can accurately detect the states of estrus, illness and the like of the cow, and improve the estrus exposure rate so as to determine the optimal hybridisation time according to the exposed estrus time, improve the conception rate of the estrus cow and realise automatic management of cow estrus monitoring.

### MEANS FOR SOLVING PROBLEM

To solve the problems above and achieve an object, a notifying method, a notifying program, and a notifying apparatus that receive a first time point at which an onset of disease is confirmed in a livestock animal among a livestock animal group, and a disease name of the disease afflicting the livestock animal; identify disease duration corresponding to the received disease name of the disease afflicting the livestock animal, by referring to a first storage unit that stores a disease name of a disease and disease duration of the disease in a correlated manner; acquire from a second storage unit that stores for respective time slots of each date, measurement results of a step count of another livestock animal different from the livestock animal in the livestock animal group, a measurement result of the other livestock animal for a first time slot that includes the first time point and a measurement result of the other livestock animal for a second time slot that includes a second time point traced back from the first time point, at least by a period of the identified disease duration; determine whether the disease afflicting the livestock animal is afflicting the other livestock animal, based on the acquired measurement result of the other livestock animal for the first time slot, the acquired measurement result of the other livestock animal for the second time slot, and a threshold value related to the step count specific to the disease afflicting the livestock animal; and output a disease name of the disease of the livestock animal and identification information of the other livestock animal, upon determination that the disease afflicting the livestock animal is afflicting the other livestock animal.

### EFFECT OF THE INVENTION

According to one aspect of the present invention, an effect is achieved in that the finding of a livestock animal that has a disease can be supported.

### BRIEF DESCRIPTION OF DRAWINGS

FIG. 1 is an explanatory view of an example of a notifying method according to an embodiment;
FIG. 2 is an explanatory view of a system configuration example of a notifying system 200;
FIG. 3 is a block diagram of a hardware configuration example of a communications device 101;
FIG. 4 is a block diagram of a hardware configuration example of a relay device 211;
FIG. 5 is a block diagram of a hardware configuration of a notifying apparatus 102, etc.;
FIG. 6 is an explanatory view of an example of storage contents of a measurement result information table 201;
FIG. 7 is an explanatory view of an example of storage contents of a step count information DB 202;
FIG. 8 is an explanatory diagram of an example of storage contents of a disease information DB 203;
FIG. 9 is an explanatory diagram of an example of storage contents of a worker information DB 204;
FIG. 10 is a block diagram of a functional configuration of the notifying apparatus 102;
FIG. 11 is an explanatory diagram of an example of a screen displayed on a client apparatus 230;
FIG. 12 is a flowchart of an example of a communication process procedure of the relay device 211;
FIG. 13 is a flowchart (part one) of an example of a notifying process procedure of the notifying apparatus 102;
FIG. 14 is a flowchart (part two) of an example of the notifying process procedure of the notifying apparatus 102;
FIG. 15 is a flowchart of an example of a disease determining process procedure of the notifying apparatus 102;
FIG. 16 is a flowchart of an example of the notifying process procedure of the client apparatus 230;
FIG. 17 is an explanatory diagram of an example of comparison of behavior between a livestock animal infected in the past and a herd at the time;
FIG. 18 is an explanatory diagram of an example of comparison of behavior between the current livestock animal A and the herd that includes the livestock animal A;
FIG. 19 is a flowchart of an example of a threshold value setting process procedure executed by the notifying apparatus 102;
FIG. 20 is an explanatory diagram of an example of storage contents of a potential infection DB 2001; and
FIG. 21 is a flowchart of an example of a disease possibility determining process procedure executed by the notifying apparatus 102.

### BEST MODE(S) FOR CARRYING OUT THE INVENTION

Embodiments of a notifying method, a notifying program, and a notifying apparatus according to the present invention will be described in detail with reference to the accompanying drawings.

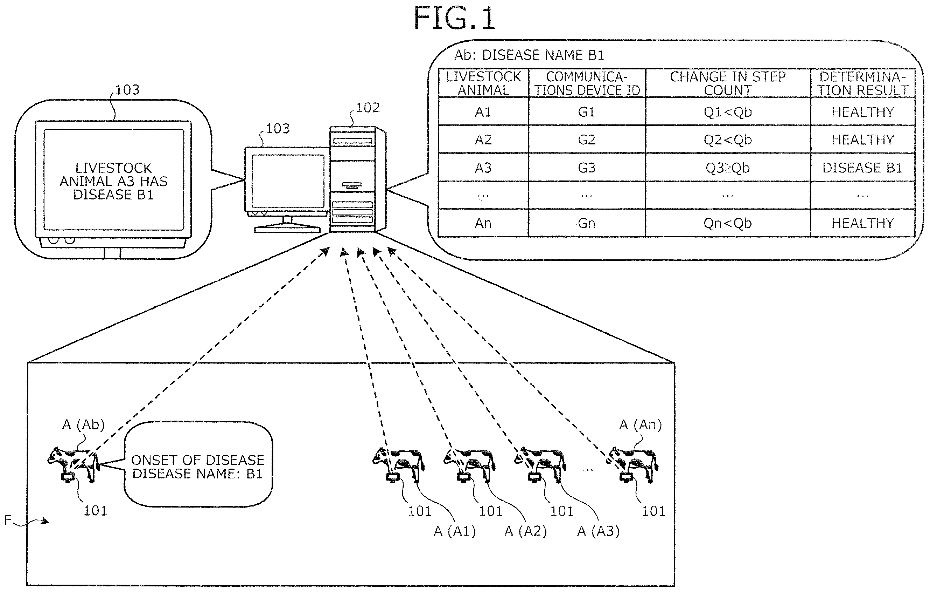

FIG. 1 is an explanatory view of an example of a notifying method according to the present embodiment. In FIG. 1, livestock animals A are raised on the premises of a farm F managed by a worker. The worker is a person engaged in livestock farming. The farm F refers to facilities including the pasture area for pasturing the livestock animals A. The livestock animals A are animals capable of moving inside the site of the farm F, for example, the pasture area of the farm F. For example, the livestock animals A may be animals moving by ambulation such as cattle, pigs, and horses.

The livestock animals A1 to An are equipped with respective communications devices 101. In this case, the communications devices 101 are portable computers that measure the step counts of the livestock animals A. For example, when walking, the livestock animals A first put out a right leg toward the ground in the direction of travel. When the right leg is planted on the ground, the livestock animals A then put out a left leg toward the ground in the direction of travel. When the left leg is planted on the ground, the livestock animals A repeat the motion such as putting out a right leg again. In particular, the step counts of the livestock animals A can be defined as the numbers of times the livestock animals A put out a right leg or a left leg toward the ground in the direction of travel.

For example, when the livestock animal A is afflicted with a disease, the step count of the livestock animal A per unit time increases or decreases as compared to when the livestock animal A is healthy. Disease refers to a poor or undesirable physical or emotional state of the livestock animal A. The onset of disease refers to occurrence of disease in the livestock animal A.

The communications device 101 can communicate with multiple relay devices disposed on the premises of the farm F and is a computer capable of communication with a notifying apparatus 102 via a corresponding relay device. The relay devices are respectively disposed at a different position on the farm F and each of the relay devices forms a communication area having a radius of 150 m, for example. The communications device 101 transmits measurement result information indicative of a measurement result and a communications device ID specific to the communications device 101 to any communicable one of the relay devices.

The notifying apparatus 102 has a display 103, and is a computer capable of receiving from the communications device 101, the measurement result information and the communications device ID of the communications device 101, via the relay device. The notifying apparatus 102 stores for each communications device ID, hourly measurement result information correspond to a predetermined period. The notifying apparatus 102 uses the measurement result information for each communications device ID to determine whether a livestock animal A in estrus is present.

It is assumed that if an onset of disease is confirmed in one livestock animal, a past measurement result is used for determining the presence/absence of the onset of disease for the other cattle by utilizing the fact that the step count of the livestock animal A changes when the livestock animal A has developed a disease. For example, when the onset of disease is confirmed in one livestock animal during a periodic health examination, for another livestock animal A, if a difference exists between a past measurement result in a healthy state and a current measurement result and is greater than or equal to a predetermined value, it is determined that the other livestock animal A has developed the disease.

Assuming that three days have already passed after the onset of disease in the other livestock animal A, a measurement result acquired four or more days before must be used as the measurement result in the healthy state for the other livestock animal A. If a measurement result within three days is used for comparison between this measurement result and the current measurement result, a difference that is greater than or equal to the predetermined value is not obtained and it may be determined that the livestock animal has no disease.

In consideration of a possibility that the disease has already developed in the near past, it is assumed that a measurement result acquired in the far past, for example, half a year ago, is used for comparison with the current measurement result. However, since the environment may be different half a year ago in terms of the number of herds or differences in terrain and food due to a change of the seasons, a large difference may generated between the past measurement result and the current measurement result. Therefore, a difference greater than or equal to the predetermined value may be obtained between the past measurement result in a healthy state and the current measurement result, and even a healthy livestock animal A may be determined as having a disease.

Since the livestock animals A are easily affected by the other livestock animals in a herd and it may be difficult to comprehend a sign of the onset of disease by simply using past statistics indicative of a decreasing tendency of the step count of each disease.

Therefore, in the embodiment, at the time of confirmation of a livestock animal A (Ab), if a difference exists between the current step count taken by the other livestock animals A1 to An and the step count traced back from the present, by a period of the disease duration of the same disease type, and the difference is the same value as the livestock animal Ab, it is determined that the other livestock animals A1 to An have developed the disease. As a result, the other livestock animals A1 to An having the same type of disease as the diseased livestock animal Ab can be found. An average value of the step count may be compared between a livestock animal A developing a disease in the past and the head including the livestock animal A to make a notification of a possibility of infection of the livestock animal A with the disease before an onset of the disease of the livestock animal A, from a difference of a comparison result at the time of infection before the onset.

An example of the notifying method of an embodiment will be described.

The communications device 101 transmits the measurement result information indicative of a communications device ID identifying a livestock animal A and the step count of the livestock animal A, via a relay device, at predetermined transmission intervals. The predetermined transmission interval is one hour, for example.

The notifying apparatus 102 receives the communications device ID and the measurement result information from the communications device 101 and stores the measurement result information of each time slot, for each communications device ID.

It is assumed that an onset of a disease with a disease name B1 is confirmed in a livestock animal Ab during a periodic health examination, etc. The worker inputs into the notifying apparatus 102, the disease name B1 of the livestock animal Ab and the time of confirmation of the onset of disease. The time of confirmation of the onset of disease is not the time when the livestock animal Ab has actually developed the disease and is the time when the worker confirms that the livestock animal Ab has developed the disease. The notifying apparatus 102 identifies disease duration of the disease name B1 and acquires information of a threshold value Hb related to the step count specific to the disease name B1. The disease duration is the duration from the onset of the disease of the disease name B1 until recovery. It is assumed that the livestock animals A do not continuously have the disease for a period longer than the disease duration. The threshold value Hb is a value for determining the disease name B1 from a measurement result of the step count.

The notifying apparatus 102 acquires a measurement result N2 of another livestock animal A (e.g., A1) for time point traced back by the disease duration of the disease name B1, and a measurement result N1 of the livestock animal A1 when the onset of disease is confirmed. For example, if the disease duration of the disease name B1 is, for example, seven days and the time point of confirmation of the onset of disease is 11:50 on February 8, the notifying apparatus 102 acquires the measurement result N2 of the livestock animal A1 obtained seven days before from 11:00-12:00 on February 1 and the measurement result N1 of the livestock animal A1 from 11:00-12:00 on February 8.

The notifying apparatus 102 uses the acquired measurement results N1, N2, and the threshold value Hb to determine whether the disease of the disease name B1 occurring in the livestock animal Ab is afflicting the other livestock animal A1. For example, the notifying apparatus 102 calculates a value such as a difference or a ratio between the measurement result N2 and the measurement result N1 and determines that the disease of the disease name B1 is afflicting the other livestock animal A1 if the calculated value is greater than or equal to the threshold value Hb.

In the embodiment, the notifying apparatus 102 determines that the disease of the disease name B1 is afflicting the livestock animal A1 if the measurement result N1 is reduced by the threshold value Hb or more as compared to the measurement result N2 in the healthy state. For example, the notifying apparatus 102 calculates "N2-N1=Q" and determines that the livestock animal A1 is healthy in the case of "Q<Hb" and determines that the disease of the disease name B1 is afflicting the livestock animal A1 in the case of "Q≥Hb".

The notifying apparatus 102 determines whether "Q" is greater than or equal to the threshold value Hb for each of the livestock animals A1 to An. For example, it is assumed that a livestock animal A3 of a communications device ID "G3" has "Q" greater than or equal to the threshold value Hb. In this case, the notifying apparatus 102 displays that the disease of the disease name B1 is afflicting the livestock animal A3 greater than or equal to the threshold value Hb.

The acquisition of the measurement result N2 for a time point traced back by the disease duration will additionally be described. For example, it is assumed that the period of disease duration is seven days. If the livestock animal A1 developed the disease of the disease name B1 six days ago, the measurement result N2 of the livestock animal A1 in the healthy state can be acquired by tracing back by at least the disease duration when the current disease of the livestock animal A1 is determined. On the other hand, for example, if the measurement result N2 is acquired for the time point traced back by a number of days, such as six days, that is less than the disease duration, the measurement result N2 after the onset of the disease may be acquired and the measurement result N2 in the healthy state cannot be used for comparing the measurement results in some cases. Therefore, in this embodiment, the measurement result N2 for the time point traced back by the disease duration is acquired.

If the livestock animal A2 developed the disease of the disease name B1 ten days ago, the measurement result N2 of the livestock animal A2 for the time point traced back by the disease duration is the result during the disease. However, since the disease duration is seven days, the livestock animal A2 has already recovered to the healthy state at the present when the onset of the disease of the disease name B1 is confirmed in the livestock animal Ab. In this case, for the livestock animal A2 having the disease for the time point for the time point traced back by the disease duration, it can be determined from a difference or a ratio between N1 and N2 that the livestock animal A2 has recovered from the disease.

According to the notifying method of the embodiment described above, at the time of confirmation of the livestock animal Ab, if a difference exists between the current step count taken by the other livestock animals A1 to An and the step count for the time point traced back by disease duration of the same disease type from the present and is the same value as the livestock animal Ab, it can be determined that the other livestock animals A1 to An have developed the disease. As a result, the other livestock animals A1 to An having the same type of disease as the diseased livestock animal Ab can be found and a possibility of infection of the other livestock animals A1 to An with the disease can be indicated.

### (System Configuration Example of Notifying System 200)

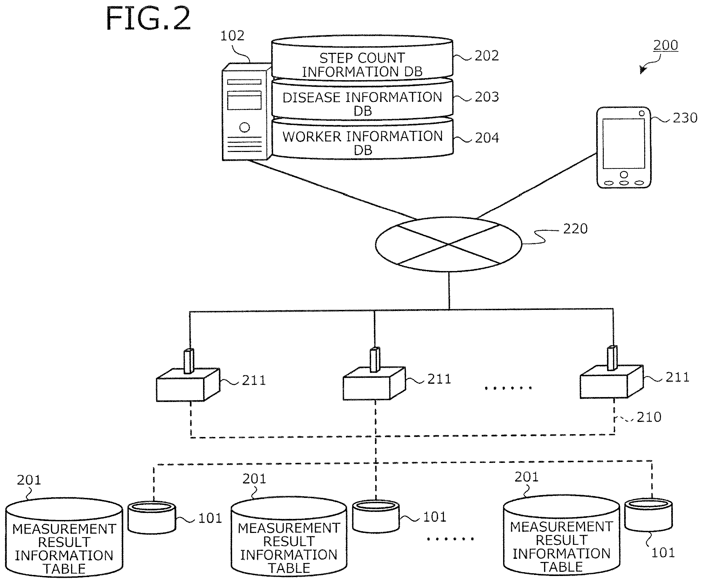

A system configuration example of a notifying system 200 will be described. FIG. 2 is an explanatory view of a system configuration example of the notifying system 200. In FIG. 2, the notifying system 200 includes the multiple communications devices 101, the notifying apparatus 102, the multiple relay devices 211, and a client apparatus 230.

In the notifying system 200, the communications devices 101 and the relay devices 211 are connected via a wireless communications network 210. Each of the communications devices 101 and the relay devices 211 has a given range around the communications device or the relay device, for example, a range of a 100-meter radius around the communications device or the relay device, as a communicable area enabling communication through the wireless communications network 210. If the communications device 101 and the relay device 211 are in a positional relationship enabling the communication, the communications device 101 and the relay device 211 are connected through the wireless communications network 210. For example, the wireless communications network 210 can be implemented by near field communication such as radio frequency identification (RFID).

The relay devices 211, the notifying apparatus 102, and the client apparatus 230 are connected via a network 220. For example, the network 220 may be the Internet, a local area network (LAN), a wide area network (WAN), etc.

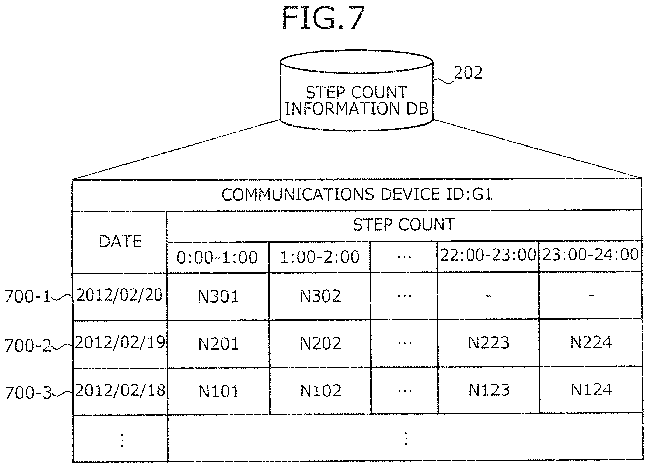

The communications devices 101 have measurement result information tables 201, etc., and are portable computers equipped to the respective livestock animals A raised in the farm F. Each of the communications devices 101 has a measurement function of measuring the step count of the livestock animal equipped with the communications device 101 and a communication function through the wireless communications network 210. For example, the communications devices 101 can be implemented by applying pedometers to which a communication function through the wireless communications network 210 is added. Storage contents of the measurement result information tables 201 will be described later with reference to FIG. 7.

The relay devices 211 are disposed on the premises of the farm F and are computers having a communication function through the wireless communications network 210 and a communication function through the network 220. The multiple relay devices 211 are respectively disposed at different installation positions.

The notifying apparatus 102 has a step count information DB 202, a disease information DB 203, a worker information DB 204, etc., and is a computer having a communication function through the network 220. For example, the notifying apparatus 102 can be implemented by applying a server included in a cloud computing system and a personal computer (PC) or a notebook PC used by a manager or a worker W of the farm F. Storage contents of the step count information DB 202, the disease information DB 203, and the worker information DB 204 will be described later with reference to FIGs. 7 to 9.

The client apparatus 230 is a computer having a communication function through the network 220. The client apparatus 230 can be implemented by applying a PC or a notebook PC, a portable telephone, and a smartphone used by a worker W of the farm F.

### (Hardware Configuration Example of Communications Device 101)

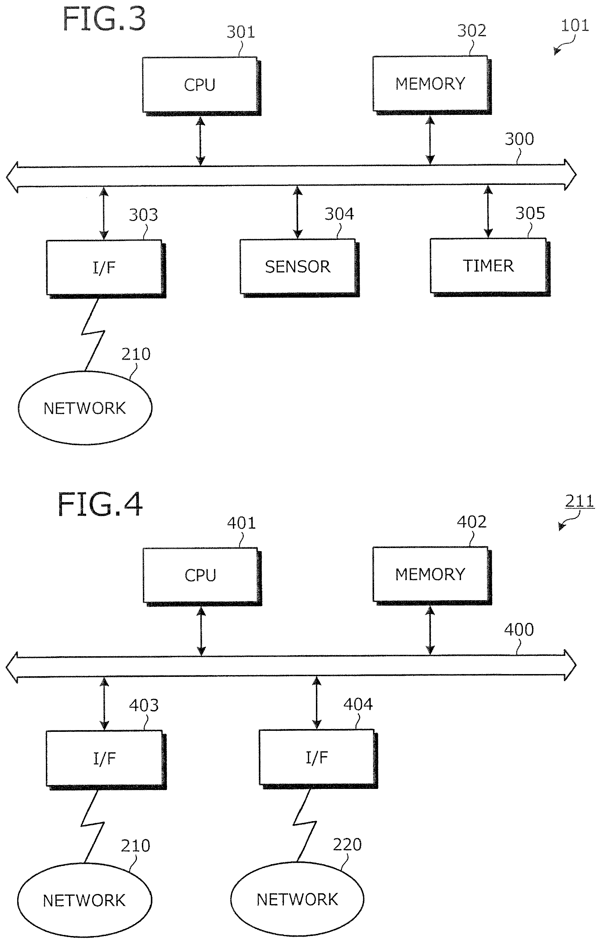

A hardware configuration example of the communications device 101 will be described. FIG. 3 is a block diagram of a hardware configuration example of the communications device 101. In FIG. 3, the communications device 101 has a central processing unit (CPU) 301, memory 302, an interface (I/F) 303, a sensor 304, and a timer 305. The constituent elements are connected to each other through a bus 300.

The CPU 301 is responsible for general control of the communications device 101. The memory 302 includes read only memory (ROM), random access memory (RAM), and flash ROM. The ROM and the flash ROM store various programs such as a boot program, for example. The RAM is used as a work area of the CPU 301.

The I/F 303 is connected through a communication line to the wireless communications network 210 and is connected via the wireless communications network 210 to another device (such as the relay device 211). The I/F 303 is responsible for an internal interface with the wireless communications network 210 and controls the input and output of data with respect to external devices.

The sensor 304 outputs information for detecting behavior of the communications device 101. For example, the sensor 304 is implemented by using a gyro sensor, a three-axis acceleration sensor, etc., and when acceleration occurs in the communications device 101, the sensor 304 outputs information corresponding to the acceleration. The sensor 304 has a function of counting steps with respect to the communications device 101.

The timer 305 has a time measuring function. For example, the timer 305 measures the actual time. The timer 305 may measure an elapsed time from a given time.

### (Hardware Configuration Example of Relay Device 211)

A hardware configuration example of the relay device 211 will be described. FIG. 4 is a block diagram of a hardware configuration example of the relay device 211. In FIG. 4, the relay device 211 has a CPU 401, memory 402, an I/F 403, and an I/F 204. The constituent elements are connected to each other through a bus 400.

The CPU 401 is responsible for general control of the relay device 211. The memory 402 includes ROM, RAM, and flash ROM. The ROM and the flash ROM store various programs such as a boot program, for example. The RAM is used as a work area of the CPU 401.

The I/F 403 is connected through a communication line to the wireless communications network 210 and is connected via the wireless communications network 210 to other devices such as the communications device 101. Further, the I/F 404 is connected through a communication line to the network 220 and is connected via the network 220 to other devices such as the notifying apparatus 102. The I/Fs 403 and 404 are responsible for an internal interface with the wireless communications network 210 and the network 220, and control the input and output of data with respect to external devices.

### (Hardware Configuration Example of Notifying Apparatus 102, etc.)

A hardware configuration example of the notifying apparatus 102 and the client device 230 will be described. In this example, the notifying apparatus 102 and the client device 230 will simply be referred to as the "notifying apparatus 102, etc."

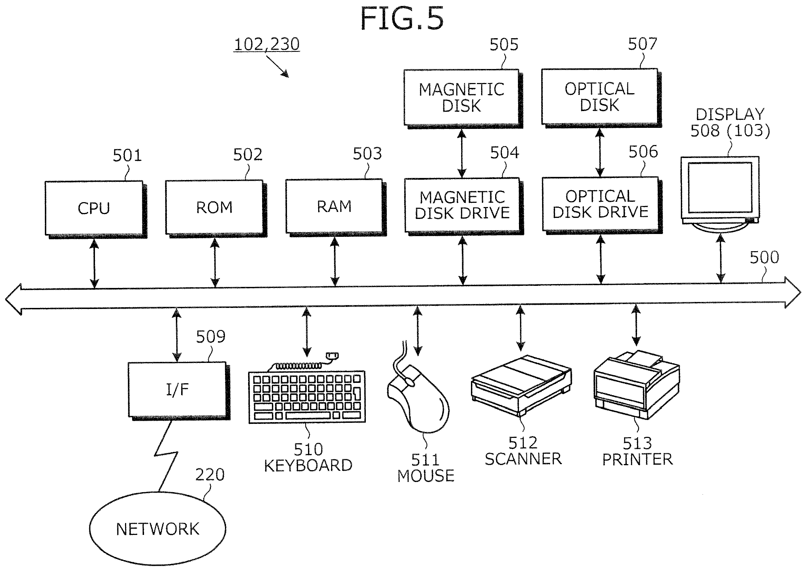

FIG. 5 is a block diagram of a hardware configuration of the notifying apparatus 102, etc. In FIG. 5, the notifying apparatus 102, etc. have a CPU 501, a ROM 502, a RAM 503, a magnetic disk drive 504, a magnetic disk 505, an optical disk drive 506, an optical disk 507, a display 508 (103), an I/F 509, a keyboard 510, a mouse 511, a scanner 512, and a printer 513. The constituent units are connected via a bus 500 to each other.

The CPU 501 governs overall control of the notifying apparatus 102, etc. The ROM 502 stores therein programs such as a boot program. The RAM 503 is used as a work area of the CPU 501. The magnetic disk drive 504, under the control of the CPU 501, controls the reading and writing of data with respect to the magnetic disk 505. The magnetic disk 505 stores therein data written under control of the magnetic disk drive 504.

The optical disk drive 506, under the control of the CPU 501, controls the reading and writing of data with respect to the optical disk 507. The optical disk 507 stores therein data written under control of the optical disk drive 506, the data being read by a computer.

The display 508 (103) displays, for example, data such as text, images, functional information, etc., in addition to a cursor, icons, and/or tool boxes. A cathode ray tube (CRT), a thin-film-transistor (TFT) liquid crystal display, a plasma display, etc., may be employed as the display 508 (103) .

The I/F 509 is connected to the network 220 through a communication line and is connected to other apparatuses (e.g., the relay devices 211 and the client apparatus 230) through the network 220. The I/F 509 administers an internal interface with the network 220 and controls the input and output of data with respect to external apparatuses. For example, a modem or a LAN adaptor may be employed as the I/F 509.

The keyboard 510 includes, for example, keys for inputting letters, numerals, and various instructions and performs the input of data. Alternatively, a touch-panel-type input pad or numeric keypad, etc. may be adopted. The mouse 511 is used to move the cursor, select a region, or move and change the size of windows. A track ball or a joy stick may be adopted provided each respectively has a function similar to a pointing device.

The scanner 512 optically reads an image and takes in the image data into the notifying apparatus 102, etc. The scanner 512 may have an optical character reader (OCR) function as well. The printer 513 prints image data and text data. The printer 513 may be, for example, a laser printer or an ink jet printer.

Further, for example, among the components described above, the notifying apparatus 102 may be configured to omit the optical disk drive 506, the optical disk 507, the display 508 (103), the mouse 511, the scanner 512, and the printer 513. The client apparatus 230 may be configured to omit the optical disk drive 506, the optical disk 507, the mouse 511, the scanner 512, and the printer 513.

### (Example of Information Stored in Communications Device 101)

An example of information stored in the communications device 101 will be described. As described above, the communications device 101 stores the measurement result information table 201. For example, the measurement result information table 201 is implemented by using the memory 302 of the communications device 101.

### <Example of Storage Contents of Measurement Result Information Table 201>

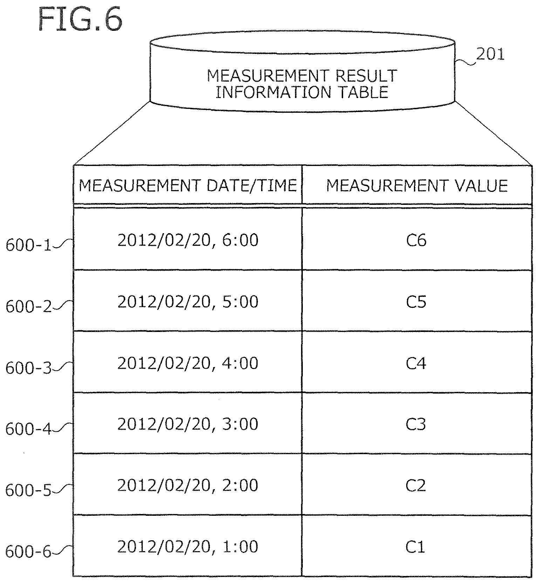

FIG. 6 is an explanatory view of an example of storage contents of the measurement result information table 201. In FIG. 6, the measurement result information table 201 has fields for measurement dates/times and measurement values. By setting information in these fields, records of measurement result information 600-1 to 600-6 are stored for respective combinations of measurement dates/times and measurement values in the measurement result information table 201.

The measurement date/time indicates date and time of transmission timing of past measurement result information. In the case of the present embodiment, by way of example, the measurement dates/times indicate dates and times of transmission timings of the measurement result information of the last six times. The measurement value indicates a measurement value of the step count of the livestock animal A at the transmission timing of past measurement result information. In the case of the present embodiment, by way of example, the measurement values indicate measurement values of the step counts of the livestock animal A at the transmission timings of the measurement result information of the last six times.

For example, the communications device 101 stores, as a current measurement value, a value acquired by accumulating the step count of the livestock animal A until the current time from a given timing such as timing of setting the measurement value to "0". Each time the livestock animal A takes one step, acceleration is momentarily generated in the communications device 101. When this acceleration is detected by the sensor 304, the communications device 101 counts up the current measurement value by "+1".

When the transmission timing of the measurement result information arrives based on the time measurement result of the time 305, the communications device 101 stores the measurement result information correlated with the current measurement information at the measurement date/time corresponding to the transmission timing. The transmission timing is on the hour, for example.

In FIG. 6, the measurement result information 600-1 indicates that the measurement value at the time point of "500 on February 20, 2012" is "C6 (C6 is a positive integer)". When the measurement result information is stored, the communications device 101 transmits the records of the measurement result information stored in the measurement result information tables 201 via the relay device 211 to the notifying apparatus 102.

Although the communications device 101 stores the measurement result information of the last six times in the described example, configuration is not limited hereto. Configuration may be such that the communications device 101 does not store past measurement result information. For example, in this case, when the transmission timing of the measurement result information arrives, the communications device 101 may transmit the current measurement value as the measurement result information and delete the measurement result information. Such a configuration can reduce a data amount stored by the communications device 101 in terms of the storage of the measurement result information.

Although the communications device 101 transmits the records of the measurement result information stored in the measurement result information tables 201 in this example, configuration is not limited hereto. For example, the communications device 101 may transmit only the current measurement result information, or particularly, only the measurement result information 600-1 in the example of FIG. 6. Such a configuration can reduce a data amount stored by the communications device 101 in terms of the transmission of the measurement result information.

The communications device 101 may transmit the current measurement result information and the measurement result information failed in transmission at past transmission timing. For example, in this case, when receiving the measurement result information from the communications device 101, the relay device 211 transmits successful reception information indicative of reception of the measurement result information to the communications device 101. If the successful reception information is not received within a given period after transmission of the measurement result information, the communications device 101 determines that the transmission of the measurement result information has failed.

In this case, the communications device 101 correlates and stores information indicative of failure of transmission with the measurement result information determined as having failed in transmission. When the transmission timing of the measurement result information subsequently arrives, the communications device 101 transmits the current measurement result information and the measurement result information failed in transmission. Such a configuration can ensure transmission of the measurement result information to the relay device 211 while reducing a data amount transmitted by the communications device 101 in terms of the transmission of the measurement result information.

### (Example of Information Stored by Notifying Apparatus 102)

An example of information stored by the notifying apparatus 102 will be described. As described above, the notifying apparatus 102 stores the step count information DB 202 and the herd management DB 203. The various DBs 202 and 203 are implemented by using a storage unit such as the ROM 502, the RAM 503, the magnetic disk 505, and the optical disk 507 of the notifying apparatus 102.

### <Example of Storage Contents of Step Count Information DB 202>

FIG. 7 is an explanatory view of an example of storage contents of the step count information DB 202. In FIG. 7, the step count information DB 202 is stored for each communications device ID and has a communications device ID for identifying the communications device 101. The step count information DB 202 has fields for dates and step counts for each time slot. By setting information in these fields, records of step-count information 700-1 to 700-3 are stored for respective combinations of dates and step count histories in the step count information DB 202.

The date indicates a date, for example, year/month/day, of measurement of the number of steps. The step count indicates the step count of the livestock animal A based on the measurement result information. The field of the step count includes a field for each time slot such as "0:00-1:00", "1:00-2:00", "2:00-3:00", ..., "22:00-23:00", and "23:00-24:00", for example. Each of the time slot fields stores information indicative of the step count of the livestock animal A during the time slot. For example, the notifying apparatus 102 stores a value acquired by subtracting a measurement value at the starting time, from a measurement value at the ending time of each of the time slot fields as the step count of the livestock animal A in the time slot into each of the time slot fields.

For example, it is assumed that the notifying apparatus 102 acquires the measurement result information including information indicating that the measurement value at "2:00 on February 20, 2012" is "C2" and that the immediately preceding measurement value at "1:00 on February 20, 2012" is "C1". In this case, the notifying apparatus 102 stores "N302 (=C2-C1)" acquired by subtracting "C1" from "C2" in the step count field identified by a date of "February 20, 2012" and a time of "2:00".

For example, the step count information 700-1 has "N301" stored in the field of "0:00-1:00" and "N302" stored in the field of "1:00-2:00", for example. The step count information is stored in the step count information DB 202 for a predetermined period such as three months, for example.

The step count field may be provided with a relay device ID field storing a relay device ID for identifying the transmission source relay device 211 when the measurement result information is received from the relay device 211 at each of the times. For example, when measurement result information is received at "2:00 on February 20, 2012", the relay device ID of the relay device 211 of the transmission source of the measurement result information may be stored in the relay device ID field. This enables understanding of a communication area in which the livestock animals A are located in each time slot. The step count information DB 202 stores the information described above for each communications device ID.

### <Example of Storage Contents of Disease Information DB 203>

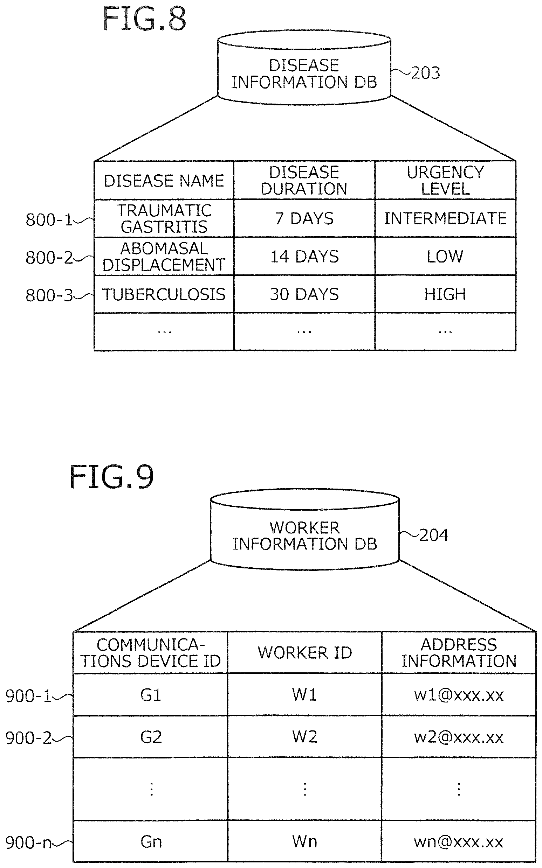

FIG. 8 is an explanatory diagram of an example of storage contents of the disease information DB 203. In FIG. 8, the disease information DB 203 has a disease name field, a disease duration field, and an urgency level field. By setting information in these fields in advance, records of disease information 800-1 to 800-3 are stored for respective combinations of disease names, disease durations, and urgency levels.

The disease name is a type of disease occurring in the livestock animals A. The disease duration is duration when each disease occurs, from the onset until the complete recovery. The disease duration is different depending on a disease name. The urgency level is a level indicative of whether an immediate treatment is required. For example, the urgency level is "low" when only medication is required, "intermediate" when treatment is required, and "high" when isolation from the other livestock animals A is required.

For example, the disease information 800-1 indicates that the disease duration of a disease name "traumatic gastritis" is "seven days" and has an urgency level of "intermediate". The disease information DB 203 stores such disease information for each disease name.

### <Example of Storage Contents of Worker Information DB 204>

FIG. 9 is an explanatory diagram of an example of storage contents of the worker information DB 204. In FIG. 9, the worker information DB 204 has a communications device ID field, a worker ID field, and an address information field. By setting information in these fields in advance, records of worker information 900-1 to 900-n are stored for respective combinations of communications device IDs, worker IDs, and address information.

The communications device IDs are identification information specific to the communications devices 101 and are information for identifying the livestock animals A. The worker IDs are identification information specific to workers for managing the livestock animals A. The workers are, for example, an owner of the livestock animals A in the case of joint management and a breeding staff breeding the livestock animals A. The address information is an e-mail address of the client apparatus 2300 owned by each worker.

For example, the worker information 900-1 indicates that a worker possessing a livestock animal A of a communications device ID "G1" has a worker ID of "W1" and address information "W1@×××.××". The worker information DB 204 stores such worker information for each communications device ID.

### (Functional Configuration Example of Notifying Apparatus 102)

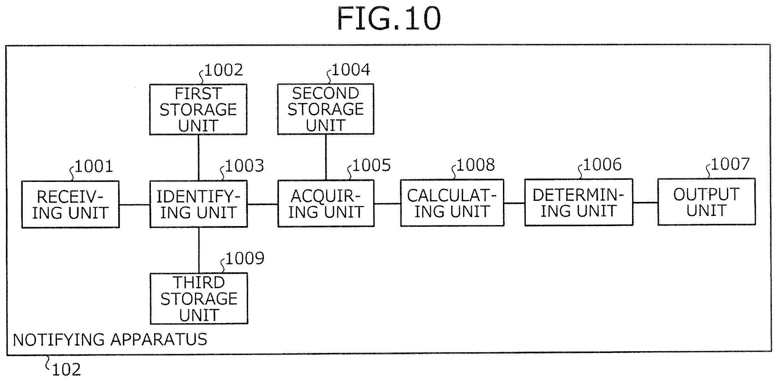

FIG. 10 is a block diagram of a functional configuration of the notifying apparatus 102. In FIG. 10, the notifying apparatus 102 includes a receiving unit 1001, a first storage unit 1002, an identifying unit 1003, a second storage unit 1004, an acquiring unit 1005, a determining unit 1006, an output unit 1007, a calculating unit 1008, and a third storage unit 1009. The functions of the functional units (the receiving unit 1002 to the third storage unit 1009) are implemented by causing the CPU 501 to execute a program stored in the magnetic disk 505 depicted in FIG. 5, for example, or by the magnetic disk 505, the I/F 509, the keyboard 510, etc. Processing results of the functional units are stored in the RAM 503, for example.

The receiving unit 1001 receives a disease name of a disease occurring in any livestock animal Ab of a livestock animal A group, and a first time point at which the onset of the disease is confirmed. Whether disease occurs in the livestock animals A is determined by, for example, periodic health examination by a veterinarian, visual observation by a worker, etc. The disease name is a type of disease occurring in the livestock animals A and may be "traumatic gastritis", "abomasal displacement", and "tuberculosis", for example. The first time point is a time point of confirmation of the onset of disease in the livestock animal Ab. The time point of confirmation of the onset of disease is a time point when it is confirmed during a periodic health examination, etc., that the already diseased livestock animal Ab has a disease rather than the time of the onset of disease.

The first storage unit 1002 stores a disease name of disease and disease duration of the disease in a correlated manner. The disease duration is the duration from the onset of disease until complete recovery and differs depending on a disease name. For example, the disease duration is defined as "7 days" for "traumatic gastritis", "14 days" for "abomasal displacement", and "30 days" for "tuberculosis". The first storage unit 1002 stores the disease information DB 203 depicted in FIG. 8, for example.

The identifying unit 1003 refers to the first storage unit 1002 to identify the disease duration corresponding to the disease name received by the receiving unit 1001. For example, if the receiving unit 1001 receives "traumatic gastritis", the identifying unit 1003 identifies "7 days" corresponding to "traumatic gastritis" as the disease duration.

The second storage unit 1004 stores measurement result information of the step counts of the other livestock animals A1 to An different from the diseased livestock animal Ab in the livestock animal A group. The second storage unit 1004 stores the measurement result information that is the step counts of the livestock animals A1 to An for respective time slots of each date within a predetermined period such as three months, for example. The second storage unit 1004 stores the step count information DB 202 depicted in FIG. 7.

The acquiring unit 1005 acquires a measurement result N1 of another livestock animal An in a first time slot including the first time point from the second storage unit 1004. The acquiring unit 1005 acquires a measurement result N2 of another livestock animal Ai (i=1 to n) in a second time slot including a second time point traced back at least by a time of the disease duration identified by the identifying unit 1003 from the first time point. In other words, the acquiring unit 1005 acquires the measurement result N2 of another livestock animal Ai in a healthy state closest to the first time point.

The measurement result N2 is described as the measurement result in a healthy state because even if the measurement result N2 of another livestock animal Ai not in a healthy state is acquired, the animal Ai is determined as being healthy at present by determination described later and is not determined as having disease.

It is assumed that the livestock animal A has developed "traumatic gastritis" at "11:50 on February 20" defined as the first time point. The first time slot is a time slot of storage of the measurement result information including the first time point and is "11:00-12:00 on February 20". The acquiring unit 1005 acquires the measurement result N1 of "11:00-12:00 on February 20" with regard to the livestock animal Ai.

The second time point is a time point traced back by the disease duration (7 days) of "traumatic gastritis" from "11:50 on February 20" defined as the first time point and is "11:50 on February 13". The second time slot is a time slot of storage of the measurement result information including the second time point and is "11:00-12:00 on February 13". The acquiring unit 1005 acquires the measurement result N1 of "11:00-12:00 on February 13" with regard to the livestock animal Ai. The first and second time slots are the same time slots with different dates. Since each of the multiple livestock animals A has a similar life pattern, the measurement results N1 and N2 in the same time slots can be compared by setting the first and second time slots as the same time slots.

For example, the determining unit 1006 determines whether a disease afflicting the livestock animal Ab is afflicting another livestock animal Ai based on the measurement result N1 of the other livestock animal Ai in the first time slot and the measurement result N2 of the other livestock animal in the second time slot acquired by the acquiring unit 1005, as well as a threshold value H. The threshold value H is a value related to the step count specific to the disease occurring in the livestock animal Ab. For example, if the livestock animal Ai has developed "traumatic gastritis", a value corresponding to the "traumatic gastritis" is used for the threshold value H.

The determining unit 1006 determines that the disease of the disease name B1 is afflicting the other livestock animal Ai if a value such as a difference or a ratio between the measurement result N1 and the measurement result N2 is greater than or equal to the threshold value H. For example, if a ratio is used, the determining unit 1006 determines that the disease is afflicting the other livestock animal Ai in the case of "(measurement result N1/measurement result N2)≥HW". Hw is a value varying depending on a disease name.

A difference is used in the embodiment and the determining unit 1006 determines that the disease is afflicting the livestock animal Ai if the measurement result N2 is reduced as compared to the measurement result N1 during a healthy state by a threshold value Hb or more. For example, the notifying apparatus 102 determines that the livestock animal A1 is healthy in the case of "N2-N1<Hb" and determines that the disease is afflicting the livestock animal A1 in the case of "N2-N1≥Hb". The threshold value Hb is a value varying depending on a disease name.

The disease may be determined as occurring not only when the difference is greater than or equal to the threshold value H but also when the difference is a value closer to the threshold value H or, for example, in the case of a disease associated with a symptom such as violent behavior, the disease may be determined as occurring when the difference is less than or equal to the threshold value H.

If the determining unit 1006 determines that the disease afflicting the livestock animal Ab is afflicting another livestock animal Ai, the output unit 1007 outputs the disease name and the identification information of the other livestock animal Ai in a correlated manner. The disease name of the other animal Ai is the same as the disease name of the livestock animal Ab confirmed as having developed the disease. The disease name and the identification information of the other livestock animal Ai are displayed on the display 103 of the notifying apparatus 120 or transmitted from the I/F 509 to the client apparatus 230 by the output unit 1007.

Since a comparison is made in the embodiment by using the past step count acquired in a healthy state in the past closest to the time of confirmation of the onset of disease in the livestock animal Ab, the comparison can accurately be performed.

The first and second time slots are desirably time slots other than during sleep and eating associated with smaller change in the step count, in view of remarkable change in the step count between a healthy state and a diseased state in a life pattern. A time point of the actual onset of disease can only be determined in units of half a day or one day rather than several hours.

From such a viewpoint, if the receiving unit 1001 receives the first time point including a time slot during eating, etc., the received first time point may be changed to a time other than the time slot during eating, etc. For example, when the time slot during eating is "12:00-13:00" and the receiving unit 1001 receives "12:15" as the first time point, the first time point may be changed to a time other than the time slot during eating, etc., for example, to 11:15 or 13:15.

The second storage unit 1004 stores a measurement result of the step count of the livestock animal Ab for each time slot. The acquiring unit 1005 acquires a measurement result N3 of the step count of the livestock animal Ab in the first time slot and a measurement result N4 of the step count of the livestock animal Ab in the second time slot.

The calculating unit 1008 calculates the threshold value H based on the measurement result N3 of the step count of the livestock animal Ab in the first time slot and the measurement result N4 of the step count of the livestock animal Ab in the second time slot acquired by the acquiring unit 1005. The calculating unit 1008 uses a difference or a ratio between the measurement result N3 and the measurement result N4 to calculate the threshold value. For example, if a ratio is used, the threshold value Hw may be calculated from "measurement result N3/measurement result N4". A calculation method of the threshold value Hw may be different depending on a disease name.

A difference is used in the embodiment and the calculating unit 1008 calculates a value indicative of how much the measurement result N3 has decreased as compared to the measurement result N4 in a healthy state. For example, the calculating unit 1008 calculates the threshold value Hb from "N4-N3". A calculation method of the threshold value Hb may be different depending on a disease name.

The determining unit 1006 determines whether a disease afflicting the livestock animal Ab is afflicting another livestock animal Ai based on the measurement result N1 of the other livestock animal Ai in the first time slot, the measurement result N2 of the other livestock animal Ai in the second time slot, and the calculated threshold value Hb. By using the threshold value Hb as described above, whether a disease afflicting the livestock animal Ab is afflicting another livestock animal Ai can properly be determined for each of the livestock animals A1 to An having similar behavior patterns in a pasture area. Particularly since the livestock animals A1 to An moving in herds have similar behavior patterns, whether a disease afflicting the livestock animal Ab is afflicting another livestock animal Ai belonging to the same herd can properly be determined.

The first storage unit 1002 stores a disease name of a disease, disease duration of the disease, and an urgency level of the disease in a correlated manner. The urgency level indicates a degree of requirement for an immediate treatment and is defined in three stages of "large, medium, and small"; however, this is not a limitation and the urgency level may be defined in two stages or four or more stages.

The identifying unit 1003 refers to the first storage unit 1002 to identify the disease duration and the urgency level corresponding to the disease name received by the receiving unit 1001. For example, if the receiving unit 1001 receives "traumatic gastritis", the identifying unit 1003 identifies "7 days" corresponding to "traumatic gastritis" as the disease duration and the urgency level "mediate".

If the determining unit 1006 determines that a disease afflicting the livestock animal Ab is afflicting another livestock animal Ai, the output unit 1007 also outputs the urgency level identified by the identifying unit 1003 in a correlated manner. As a result, a worker can clearly be notified of whether the disease occurring in the other livestock animal Ai is a disease that should immediately be treated.

The third storage unit 1009 stores addresses of communication devices of workers raising the respective livestock animals A in the livestock animal A group. For example, the third storage unit 1009 stores communications device IDs, worker IDs, and address information and stores the worker information DB 204 depicted in FIG. 9, for example. The communication devices are the client apparatus 230, for example.

If the determining unit 1006 determines that a disease afflicting the livestock animal Ab is afflicting another livestock animal Ai, the identifying unit 1003 refers to the third storage unit to identify an address of the communication device of the worker raising the other livestock animal Ai. The output unit 1007 outputs the disease name, the urgency level, and the identification information of the other livestock animal Ai to the address identified by the identifying unit 1003. As a result, the worker managing the livestock animal Ai can be notified of the onset of disease in the livestock animal Ai. The communication device is desirably a mobile communication device such as a portable telephone and a smartphone owned by the worker because the worker can promptly be notified.

### (Example of Screen Displayed on Client Apparatus 230)

An example of a screen displayed on the client apparatus 230 will be described.

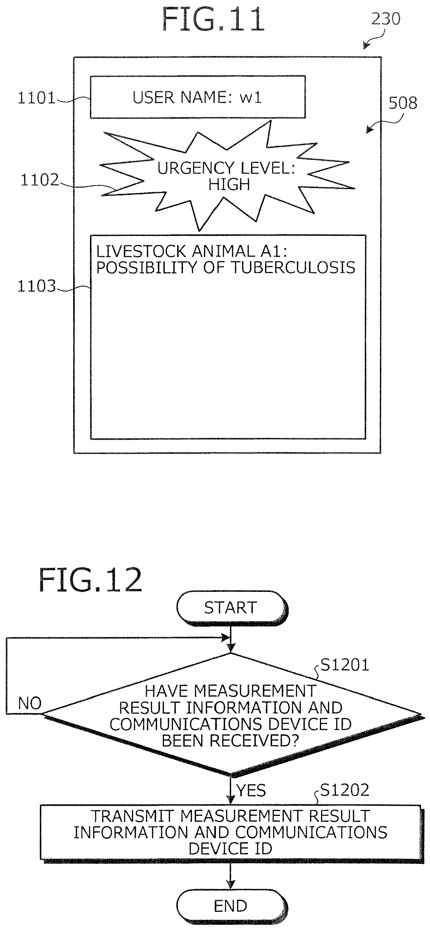

FIG. 11 is an explanatory diagram of an example of a screen displayed on the client apparatus 230. In FIG. 11, the display 508 displays a user name 1101, an urgency level 1102, and a disease name 1103. The user name 1101 is a name of a worker. The urgency level 1102 corresponds to the disease name 1103 and is displayed as "large". The disease name 1103 is displayed to indicate that the livestock animal A1 managed by the worker 1101 may have developed tuberculosis. Such a display screen can notify the worker managing the livestock animals A of the onset of disease in the livestock animal Ai when the livestock animal Ai has developed a disease, and the worker can immediately take measures such as isolating the livestock animal Ai, for example.

### (Communication Process Procedure of Relay Device 211)

A communication process procedure of the relay device 211 will be described. FIG. 12 is a flowchart of an example of the communication process procedure of the relay device 211. In the flowchart of FIG. 12, the relay device 211 determines whether measurement result information and a communications device ID have been received from the communications device 101 (step S1201). The measurement result information and the communications device ID are transmitted from the communications device 101 at intervals of one hour, for example.

The relay device 211 waits until measurement result information and a communications device ID have been received (step S1201: NO). When measurement result information and a communications device ID have been received (step S1201: YES), the relay device 211 transmits the received measurement result information and the communications device ID to the notifying apparatus 102 (step S1202) and terminates a series of operations of the present flowchart.

As described above, if the measurement result information and the communications device ID are received, the relay device 211 can transmit these records of information to the notifying apparatus 102.

### (Notifying Process Procedure of Notifying Apparatus 102)

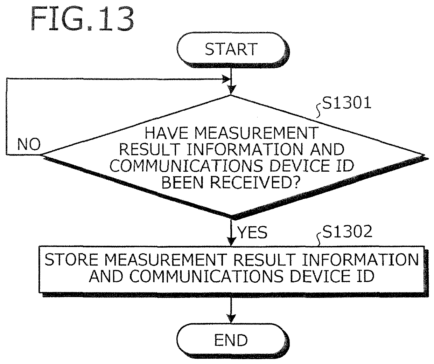

An notifying process procedure of the notifying apparatus 102 will be described. FIG. 13 is a flowchart (part one) of an example of the notifying process procedure of the notifying apparatus 102. In the flowchart of FIG. 13, the notifying apparatus 102 first determines whether measurement result information and a communications device ID of a communications device 101 have been received from the relay device 211 (step S1301). The notifying apparatus 102 waits until the measurement result information and the communications device ID have been received (step S1301: NO). The measurement result information and the communications device ID are transmitted from the relay device 211 at intervals of one hour, for example.

When the measurement result information and the communications device ID have been received (step S1301: YES), the notifying apparatus 102 stores the measurement result information and the communications device ID into the step count information DB 202 (see FIG. 7) (step S1302), and ends a series of operations according to the present flowchart. Thus, the notifying apparatus 102 can store for each predetermined period, measurement result information and a communications device ID.

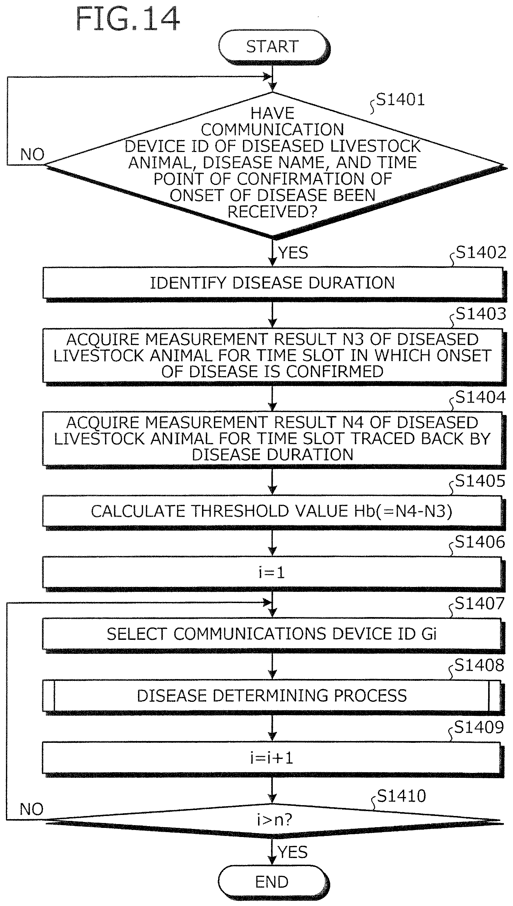

FIG. 14 is a flowchart (part two) of an example of the notifying process procedure of the notifying apparatus 102. In the flowchart of FIG. 14, the notifying apparatus 102 determines whether a communication device ID of the diseased livestock animal Ab, a disease name, and a time point of confirmation of the onset of disease have been received from a worker, for example (step S1401). The notifying apparatus 102 waits until a communication ID of the diseased livestock animal Ab, a disease name, and a time point of confirmation of the onset of disease have been received (step S1401: NO). When a communication ID of the diseased livestock animal Ab, a disease name, and a time point of confirmation of the onset of disease have been received from a worker (step S1401: YES), the notifying apparatus 102 refers to the disease information DB 203 from the received disease name to identify the disease duration (step S1402).

The notifying apparatus 102 refers to the step count information DB 202 to acquire the measurement result N3 of the diseased livestock animal Ab for the time slot in which the onset of disease is confirmed (step S1403). The notifying apparatus 102 refers to the step count information DB 202 to acquire the measurement result N4 of the diseased livestock animal Ab for the time slot traced back by the disease duration (step S1404). The measurement result N4 indicates the step count in a healthy state.

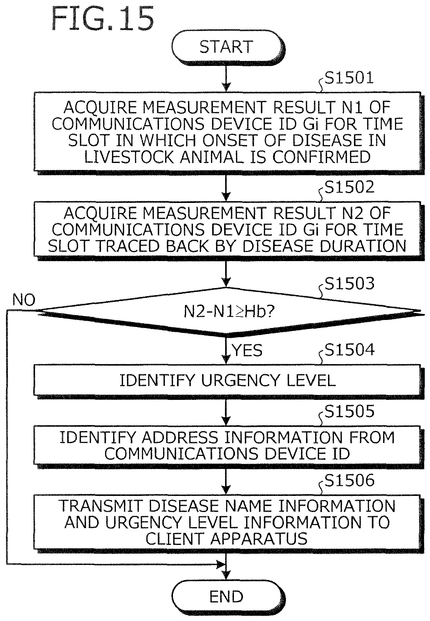

The notifying apparatus 102 subtracts measurement result N3 from the measurement result N4 to calculate the threshold value Hb (step S1405). The notifying apparatus 102 sets "1" as "i" indicative of a value of "1 to n" of the communications device IDs "G1" to "Gn" (step S1406). The notifying apparatus 102 selects the communications device ID "Gi" (step S1407). The notifying apparatus 102 executes a disease determining process (step S1408). Details of the disease determining process will be described later with reference to FIG. 15.

The notifying apparatus 102 adds "1" to "i" (step S1409) and determines whether "i" is greater than "n" (step S1410). If "i" is less than or equal to "n" (step S1410: NO), the notifying apparatus 102 transitions to the operation at step S1407. If "i" is greater than "n" (step S1410: YES), the notifying apparatus 102 terminates a series of operations of the present flowchart. As a result, the notifying apparatus 102 can calculate the threshold value Hb and can select measurement results of each of the communications devices 101 to define an object communications device ID as a determination object of disease setting.

The disease determining process procedure depicted at step S1408 of FIG. 14 will be described with reference to FIG. 15. FIG. 15 is a flowchart of an example of the disease determining process procedure of the notifying apparatus 102. In the flowchart of FIG. 15, the notifying apparatus 102 refers to the step count information DB 202 to acquire the measurement result N1 of the communications device ID "Gi" for the time slot in which the onset of disease in the livestock animal Ab is confirmed (step S1501). The notifying apparatus 102 acquires the measurement result N2 of the communications device ID "Gi" for the time slot traced back by the disease duration (step S1502). The measurement result N2 indicates the step count in a healthy state.

The notifying apparatus 102 determines if a value acquired by subtracting measurement result N1 from the measurement result N2 is greater than or equal to the threshold value Hb (step S1503). If a value acquired by subtracting measurement result N1 from the measurement result N2 is not greater than or equal to the threshold value Hb (step S1503: NO), the notifying apparatus 102 terminates a series of operations of the present flowchart. If a value acquired by subtracting measurement result N1 from the measurement result N2 is greater than or equal to the threshold value Hb (step S1503: YES), the notifying apparatus 102 refers to the disease information DB 203 to identify the urgency level (step S1504).

The notifying apparatus 102 refers to the worker information DB 204 to identify the address information from the communications device ID (step S1505). The notifying apparatus 102 transmits disease name information and urgency level information to the client apparatus 230 (step S1506) and terminates a series of operations of the present flowchart.

As a result, if the onset of disease in the livestock animal Ab is confirmed, determination can be made for the other livestock animals Ai to decide another livestock animal Ai having developed the same disease as the livestock animal Ab can be determined from a difference in the step count between the time point traced back by the disease duration defined by the disease name of the livestock animal Ab and the time point of confirmation in the livestock animal Ab. As a result, the onset of disease in another livestock animal Ai can be found earlier.

### (Notifying Process Procedure of Client Apparatus 230)

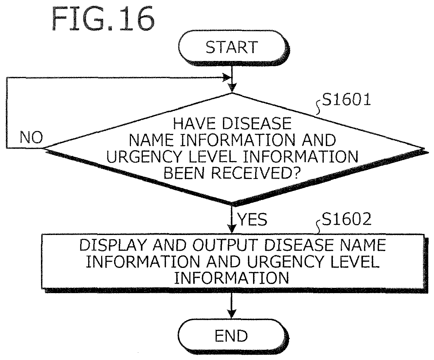

A notifying process procedure of the client apparatus 230 will be described. FIG. 16 is a flowchart of an example of the notifying process procedure of the client apparatus 230. In the flowchart of FIG. 16, the client apparatus 230 determines whether the disease name information and the urgency level information have been received from the notifying apparatus 102 (step S1601).

The client apparatus 230 waits until the disease name information and the urgency level information have been received (step S1601: NO). When the disease name information and the urgency level information have been received (step S1601: YES), the client apparatus 230 displays and outputs the disease name information and the urgency level information (step S1602) and terminates a series of operations of the present flowchart.

As a result, if the livestock animal Ai has developed a disease, the worker managing the livestock animal Ai can be notified of the onset of disease in the livestock animal Ai. For example, different ringtones or alarm sounds may be used depending on the urgency level and, even when a worker is engaged in another work, such a configuration allows the worker to suspend the work to check details of notification if the urgency level is high.

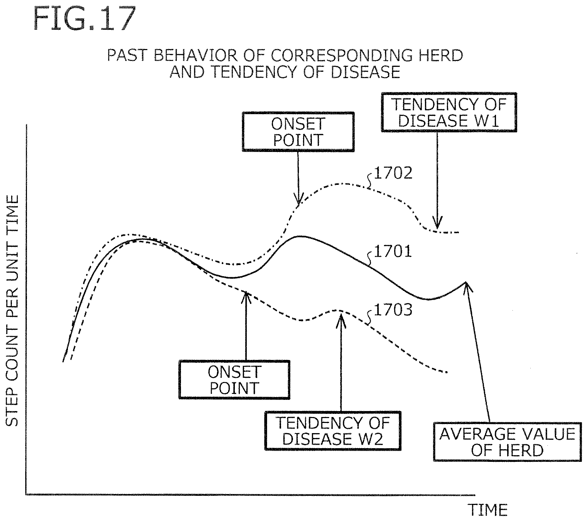

With reference to FIGs. 17 to 21, description will be made of a configuration in which average values of the numbers of steps are compared between a livestock animal A that developed a disease in the past and a herd including the livestock animal A to enable detection of possibility of infection of the livestock animal A with the disease from a difference of the comparison result at the time point of infection before the onset of disease.

### (Example of Comparison of Behavior Between Livestock Animal A Infected in the Past and Herd at the Time)

An example of comparison of behavior between a livestock animal A infected in the past and a herd at the time will be described with reference to FIG. 17. FIG. 17 is an explanatory diagram of an example of comparison of behavior between a livestock animal infected in the past and a herd at the time. In FIG. 17, the vertical axis indicates the step count per unit time and the horizontal axis indicates elapsed time. A solid line 1701 indicates an average value in the livestock animals A in the herd except the livestock animals A in estrus increasing the step count. Increase and decrease in the step count are due to weather and terrain, for example.

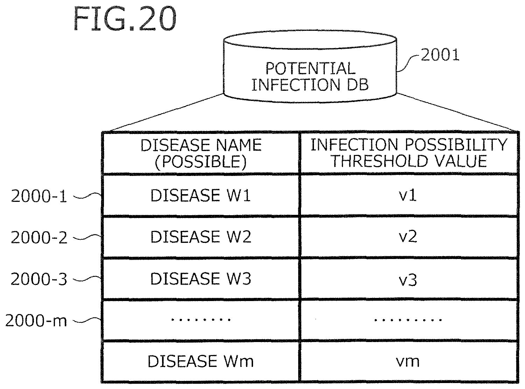

A dashed-dotted line 1702 indicates variations in the step count when the certain livestock animal A developed a disease W1. A dotted line 1703 indicates variations in the step count when the certain livestock animal A developed a disease W2. Calculation of a threshold value of the step count for each disease will be described. First, if the onset of the disease W1 is confirmed, an influence point can be identified as a time point acquired by counting back an incubation period preset for each disease from the timing of the onset of disease. A threshold value of the disease W1 is defined as a value acquired by multiplying a difference between the step count at the influence point and the average value by a safety factor of 1.1 to 1.2, for example. A threshold value is also calculated for the disease W2 in the same way. The threshold values of the respective diseases calculated as described above are stored in an potential infection DB depicted in FIG. 20.

### (Example of Comparison of Behavior between Current Livestock Animal and Herd Including Livestock animal)

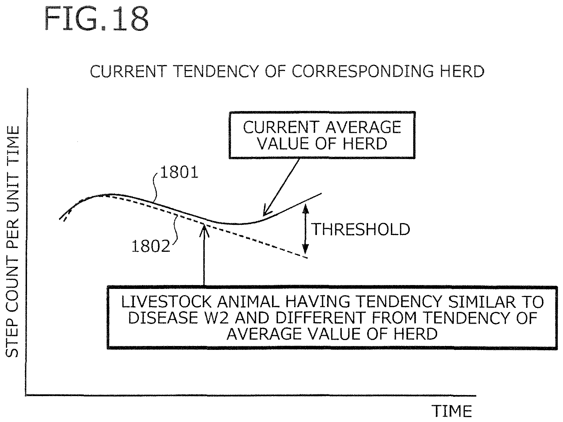

FIG. 18 is an explanatory diagram of an example of comparison of behavior between the current livestock animal A and the herd that includes the livestock animal A. In FIG. 18, the vertical axis indicates the step count per unit time and the horizontal axis indicates elapsed time. A solid line 1801 indicates an average value in the livestock animals A in the herd except the livestock animals A in estrus increasing the step count. A dotted line 1802 indicates variations in the step count of the determination object livestock animal A. If a difference between the step count of the determination object livestock animal A and the average value becomes greater than or equal to the threshold value of, for example, the disease W2, it can be determined that the livestock animal A is infected with the disease W2.

### (Threshold Value Setting Process Procedure of Notifying Apparatus 102)

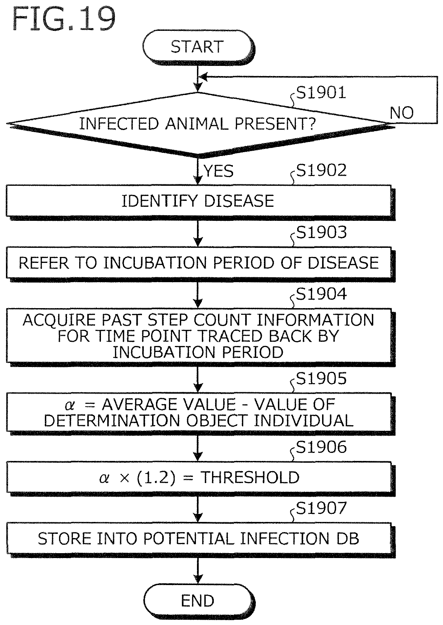

FIG. 19 is a flowchart of an example of a threshold value setting process procedure executed by the notifying apparatus 102. In the flowchart of FIG. 19, the notifying apparatus 102 waits until receiving from, for example, a worker who confirms an infected animal, input indicative of whether an infected livestock animal is present (step S1901: NO). When an infected animal is present (step S1901: YES), the notifying apparatus 102 identifies the disease (step S1902). The disease is identified based on input of the worker, for example.

The notifying apparatus 102 uses a present incubation period DB not depicted to refer to the incubation period of the disease (step S1903). The notifying apparatus 102 acquires past step count information for the time point traced back by the incubation period (step S1904). The notifying apparatus 102 calculates α acquired by subtracting a value of the step count of a determination object individual from the average value (step S1905). The notifying apparatus 102 acquired the threshold value by multiplying α by the safe factor of 1.2 (step S1906), stores the threshold value into a potential infection DB 2001 (step S1907), and terminates a series of operations of the present flowchart.

### (Example of Storage Contents of Potential Infection DB 2001)

An example of storage contents of the potential infection DB 2001 stored in the notifying apparatus 102 will be described with reference to FIG. 20. FIG. 20 is an explanatory diagram of an example of storage contents of the potential infection DB 2001. In FIG. 20, the potential infection DB 2001 has a disease name field and an infection possibility threshold value field. By setting information in these fields, records of threshold information 2000-1 to 2000-m are stored for respective combinations of disease names and infection possibility threshold values in the potential infection DB 2001.

The disease name field stores disease names of W1 to Wm, for example. The infection possibility threshold value field stores a threshold value calculated in the threshold value setting process of FIG. 19. For example, in FIG. 20, the threshold information 2000-1 indicates that if the threshold value becomes greater than or equal to W1, a possibility of infection with the disease W1 exists.

### (Disease Possibility Determining Process Procedure of Notifying Apparatus 102)

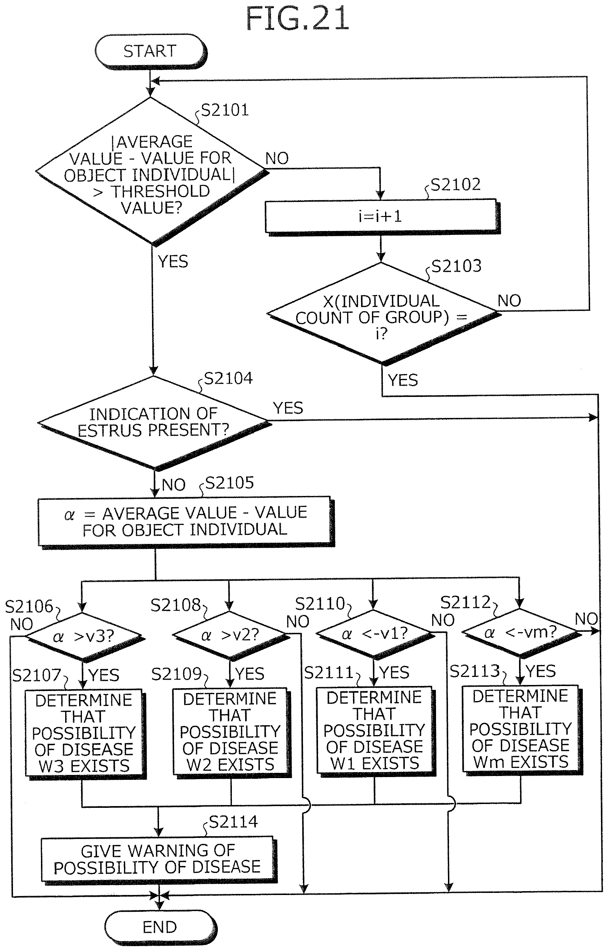

FIG. 21 is a flowchart of an example of a disease possibility determining process procedure executed by the notifying apparatus 102. In the flowchart of FIG. 21, the notifying apparatus 102 determines whether an absolute value acquired by subtracting a value of the step count of a determination object individual from the average value is greater than or equal to the threshold value (step S2101). If the absolute value is less than the threshold value (step S2101: NO), the notifying apparatus 102 sets "1" as "i" indicative of the number of individuals (step S2102). The notifying apparatus 102 determines whether i is the same number as the total number X of the individuals (step S2103).

If i is the same number as the total number X of the individuals (step S2103: YES), the notifying apparatus 102 terminates a series of operations of the present flowchart. If i is not the same number as the total number X of the individuals (step S2103: NO), the notifying apparatus 102 transitions to step S2101. If an absolute value acquired by subtracting a value of the step count of the determination object individual from the average value is greater than or equal to the threshold value (step S2101: YES), the notifying apparatus 102 determines the presence/absence of an indication of estrus of the determination object individual (step S2104). The presence/absence of a sign of estrus can be determined from, for example, the step count and, in particular, it can be determined that the individual is in estrus if the step count is greater than or equal to a predetermined value.

If the determination object individual is in estrus (step S2104: YES), the notifying apparatus 102 terminates a series of operations of the present flowchart. If the determination object individual is not in estrus (step S2104: NO), the notifying apparatus 102 calculates α acquired by subtracting a value of the step count of the determination object individual from the average value (step S2105). The notifying apparatus 102 refers to the potential infection DB 2001 of FIG. 20 to determine whether α exceeds v3 (step S2106). If α is less than or equal to v3 (step S2106: NO), the notifying apparatus 102 terminates a series of operations of the present flowchart.

If α exceeds v3 (step S2106: YES), the notifying apparatus 102 determines that a possibility of the disease W3 exists (step S2107) and transitions to the operation at step S2114. If α is less than or equal to v2 (step S2108: NO), the notifying apparatus 102 terminates a series of operations of the present flowchart. If α exceeds v2 (step S2108: YES), the notifying apparatus 102 determines that a possibility of the disease W2 exists (step S2109) and transitions to the operation at step S2114.

If α is greater than or equal to -v1 (step S2110: NO), the notifying apparatus 102 terminates a series of operations of the present flowchart. If α is less than -v1 (step S2110: YES), the notifying apparatus 102 determines that a possibility of the disease W1 exists (step S2111) and transitions to the operation at step S2114. If α is greater than or equal to -vm (step S2112: NO), the notifying apparatus 102 terminates a series of operations of the present flowchart. If α is less than -vm (step S2112: YES), the notifying apparatus 102 determines that a possibility of the disease Wm exists (step S2113), gives warning of the possibility of disease (S2114), and terminates a series of operations of the present flowchart.

The process described above enables notification of the livestock animal A having a possibility of disease, early detection of disease, and prevention of infection to the other livestock animals A.

As described above, according to the notifying apparatus 102 of the embodiment, at the time of confirmation of a livestock animal Ab, if a difference exists between the current step count taken by another livestock animal Ai and the step count for the time point traced back by disease duration of the same type of disease from the present and is the same value as the livestock animal Ab, it can be determined that the other livestock animal Ai has developed the disease. As a result, the other livestock animal Ai can be found that has developed the same type of disease as the diseased livestock animal Ab.

According to the notifying apparatus 102 of the embodiment, the threshold value H can be calculated based on the measurement result N3 of the diseased livestock animal Ab at the time of confirmation of the diseased livestock animal Ab and the measurement result N4 of the livestock animal Ab for time point traced back by the disease duration defined by the disease name of the livestock animal Ab. Therefore, a characteristic of similar behavior patterns of the livestock animals Ab, Ai in the same a pasture area can be utilized. In particular, since variations in the step count of the livestock animal Ab and the livestock animal Ai are similar, the accuracy can be improved in determination of whether the livestock animal Ai has developed the disease.

The livestock animals Ab, A1 to An moving in herds have similar behavior patterns. Therefore, the accuracy of determination can further be improved in determination of whether a disease afflicting the livestock animal Ab is afflicting another livestock animal Ai belonging to the same herd.

According to the notifying apparatus 102 of the embodiment, if it is determined that a disease afflicting the livestock animal Ab is afflicting another livestock animal Ai, the urgency level is output in a correlated manner. As a result, a worker can know the urgency level of the disease of the other livestock animal Ai. Therefore, the worker can consider whether immediate treatment is required and can take measures such as medical treatment and caring of the diseased livestock animal A and prevention of infection to the other livestock animals A, thereby suppressing a loss of the farm F.

According to the notifying apparatus 102 of the embodiment, the disease name, the urgency level, and the identification information of another livestock animal Ai can be transmitted in a correlated manner to the address associated with the communications device ID. As a result, even if different workers manage the respective livestock animals A1 to An as in the case of joint management etc., only the worker managing the livestock animal Ai can be notified of the onset of the disease. This notification allows the worker to know the urgency level of the disease of the livestock animal Ai.

The notifying method described in the present embodiment may be implemented by executing a prepared program on a computer such as a personal computer and a workstation. The notifying program is stored on a computer-readable recording medium such as a hard disk, a flexible disk, a CD-ROM, an MO, and a DVD, read out from the computer-readable medium, and executed by the computer. The notifying program may be distributed through a network such as the Internet.

### EXPLANATIONS OF LETTERS OR NUMERALS

- 101: communications device
- 102: notifying apparatus
- 211: relay device
- 202: step count information DB
- 203: disease information DB
- 204: worker information DB
- 230: client apparatus
- 1001: receiving unit
- 1002: first storage unit 1002
- 1003: identifying unit
- 1004: second storage unit
- 1005: acquiring unit
- 1006: determining unit
- 1007: output unit
- 1008: calculating unit
- 1009: third storage unit
- 2001: potential infection DB

## Claims

1. A notifying method executed by a computer, the notifying method comprising:
receiving a first time point at which an onset of disease is confirmed in a livestock animal among a livestock animal group, and a disease name of the disease afflicting the livestock animal;
identifying disease duration corresponding to the received disease name of the disease afflicting the livestock animal, by referring to a first storage unit (1002) that stores a disease name of a disease and disease duration of the disease in a correlated manner;
acquiring from a second storage unit (1004) that stores for respective time slots of each date, measurement results of a step count of another livestock animal different from the livestock animal in the livestock animal group, a measurement result of the other livestock animal for a first time slot that includes the first time point and a measurement result of the other livestock animal for a second time slot that includes a second time point traced back from the first time point, at least by a period of the identified disease duration;
determining whether the disease afflicting the livestock animal is afflicting the other livestock animal, based on the acquired measurement result of the other livestock animal for the first time slot, the acquired measurement result of the other livestock animal for the second time slot, and a threshold value related to the step count specific to the disease afflicting the livestock animal; and
outputting a disease name of the disease of the livestock animal and identification information of the other livestock animal, upon determination that the disease afflicting the livestock animal is afflicting the other livestock animal.

2. The notifying method according to claim 1, wherein
the second storage unit (1004) further stores measurement results of the step count of the livestock animal for the respective time slots,
the acquiring includes acquiring from the second storage unit (1004), a measurement result of the step count of the livestock animal for the first time slot and a measurement result of the step count of the other livestock animal for the second time slot,
the notifying method further comprising
calculating the threshold value based on the acquired measurement result of the step count of the livestock animal for the first time slot and the acquired measurement result of the step count of the other livestock animal for the second time slot, and
the determining includes determining whether a disease afflicting the livestock animal is afflicting the other livestock animal, based on the measurement result of the other livestock animal for the first time slot, the measurement result of the other livestock animal for the second time slot, and the calculated threshold value.

3. The notifying method according to claim 1 or 2, wherein
the first storage unit (1002) stores a disease name of a disease, disease duration of the disease, and an urgency level of the disease in a correlated manner,
the identifying includes identifying a disease duration and an urgency level that correspond to the received disease name of the disease afflicting the livestock animal, by referring to the first storage unit (1002), and
the outputting includes outputting the identified urgency level in a correlated manner, upon determination that the disease afflicting the livestock animal is afflicting the other livestock animal.

4. The notifying method according to claim 3, wherein
the identifying includes identifying an address of a communication device of a worker raising the other livestock animal, upon determination that the disease afflicting the livestock animal is afflicting the other livestock animal, the address being identified by referring to a third storage unit that stores an address of a communication device of a worker raising each livestock animal in the livestock animal group, and
the outputting includes outputting in a correlated manner to the identified address, the disease name of the disease afflicting the livestock animal, the identified urgency level, and identification information of the other livestock animal.

5. A notifying program executed by a computer, the notifying program causing the computer to execute a process comprising:
receiving a first time point at which an onset of disease is confirmed in a livestock animal among a livestock animal group, and a disease name of the disease afflicting the livestock animal;
identifying disease duration corresponding to the received disease name of the disease afflicting the livestock animal, by referring to a first storage unit (1002) that stores a disease name of a disease and disease duration of the disease in a correlated manner;
acquiring from a second storage unit (1004) that stores for respective time slots of each date, measurement results of a step count of another livestock animal different from the livestock animal in the livestock animal group, a measurement result of the other livestock animal for a first time slot that includes the first time point and a measurement result of the other livestock animal for a second time slot that includes a second time point traced back from the first time point, at least by a period of the identified disease duration;
determining whether the disease afflicting the livestock animal is afflicting the other livestock animal, based on the acquired measurement result of the other livestock animal for the first time slot, the acquired measurement result of the other livestock animal for the second time slot, and a threshold value related to the step count specific to the disease afflicting the livestock animal; and
outputting a disease name of the disease of the livestock animal and identification information of the other livestock animal, upon determination that the disease afflicting the livestock animal is afflicting the other livestock animal.

6. A notifying apparatus (102) comprising:
a receiving unit (1001) that receives a first time point at which an onset of disease is confirmed in a livestock animal among a livestock animal group, and a disease name of the disease afflicting the livestock animal;
an identifying unit (1003) that identifies disease duration corresponding to the received disease name of the disease afflicting the livestock animal, by referring to a first storage unit (1002) that stores a disease name of a disease and disease duration of the disease in a correlated manner;
an acquiring unit (1005) that acquires from a second storage unit (1004) that stores for respective time slots of each date, measurement results of a step count of another livestock animal different from the livestock animal in the livestock animal group, a measurement result of the other livestock animal for a first time slot that includes the first time point and a measurement result of the other livestock animal for a second time slot that includes a second time point traced back from the first time point, at least by a period of the disease duration identified by the identifying unit (1003);
a determining unit (1006) that determines whether the disease afflicting the livestock animal is afflicting the other livestock animal, based on the measurement result of the other livestock animal for the first time slot and the measurement result of the other livestock animal for the second time slot acquired by the acquiring, and a threshold value related to the step count specific to the disease afflicting the livestock animal; and
an output unit (1007) that output a disease name of the disease of the livestock animal and identification information of the other livestock animal, upon determination, by the determining unit (1006), that the disease afflicting the livestock animal is afflicting the other livestock animal.

## Patentansprüche

1. Benachrichtigungsverfahren, das von einem Computer ausgeführt wird, wobei das Benachrichtigungsverfahren Folgendes umfasst:
Empfangen eines ersten Zeitpunkts, zu dem ein Ausbruch einer Krankheit bei einem Nutztier aus einer Nutztiergruppe bestätigt wird, und eines Krankheitsnamen der Krankheit, die das Nutztier belastet;
Identifizieren einer Krankheitsdauer, die dem empfangenen Krankheitsnamen der Krankheit, die das Nutztier belastet, entspricht, durch Bezugnehmen auf eine erste Speichereinheit (1002), die einen Krankheitsnamen einer Krankheit und eine Krankheitsdauer der Krankheit auf korrelierte Weise speichert;
Beziehen von Messergebnissen einer Schrittzahl eines anderen Nutztiers, das sich von dem Nutztier in der Nutztiergruppe unterscheidet, von einer zweiten Speichereinheit (1004), die jedes Datum für jeweilige Zeitfenster speichert, einem Messergebnis des anderen Nutztiers für ein erstes Zeitfenster, das den ersten Zeitpunkt beinhaltet, und einem Messergebnis des anderen Nutztiers für ein zweites Zeitfenster, das einen zweiten Zeitpunkt beinhaltet, der von dem ersten Zeitpunkt um mindestens einen Zeitraum der identifizierten Krankheitsdauer zurückverfolgt wurde;
Bestimmen, ob die Krankheit, die das Nutztier belastet, das andere Nutztier belastet, auf der Basis des bezogenen Messergebnisses des anderen Nutztiers für das erste Zeitfenster, des bezogenen Messergebnisses des anderen Nutztiers für das zweite Zeitfenster und eines Grenzwerts, der sich auf die Schrittzahl bezieht, die für die Krankheit spezifisch ist, die das Nutztier belastet; und
Ausgeben von einem Krankheitsnamen der Krankheit des Nutztiers und Identifikationsinformationen des anderen Nutztiers nach Bestimmung, dass die Krankheit, die das Nutztier belastet, das andere Nutztier belastet.

2. Benachrichtigungsverfahren nach Anspruch 1, wobei
die zweite Speichereinheit (1004) weiterhin Messergebnisse der Schrittzahl des Nutztiers für die jeweiligen Zeitfenster speichert,
das Beziehen ein Beziehen eines Messergebnisses der Schrittzahl des Nutztiers für das erste Zeitfenster und eines Messergebnisses der Schrittzahl des anderen Nutztiers für das zweite Zeitfenster von der zweiten Speichereinheit (1004) beinhaltet,
das Benachrichtigungsverfahren weiterhin Folgendes umfasst:
Berechnen des Grenzwerts auf der Basis des bezogenen Messergebnisses der Schrittzahl des Nutztiers für das erste Zeitfenster und des bezogenen Messergebnisses der Schrittzahl des anderen Nutztiers für das zweite Zeitfenster, und
das Bestimmen ein Bestimmen, ob eine Krankheit, die das Nutztier belastet, das andere Nutztier belastet, auf der Basis des Messergebnisses des anderen Nutztiers für das erste Zeitfenster, des Messergebnisses des anderen Nutztiers für das zweite Zeitfenster und des berechneten Grenzwerts beinhaltet.

3. Benachrichtigungsverfahren nach Anspruch 1 oder 2, wobei
die erste Speichereinheit (1002) einen Krankheitsnamen einer Krankheit, eine Krankheitsdauer der Erkrankung und eine Dringlichkeitsstufe der Krankheit auf korrelierte Weise speichert,
das Identifizieren ein Identifizieren einer Krankheitsdauer und einer Dringlichkeitsstufe, die dem empfangenen Krankheitsnamen der Krankheit, die das Nutztier belastet, entsprechen, durch Bezugnehmen auf die erste Speichereinheit (1002) beinhaltet und
das Ausgeben ein Ausgeben der identifizierten Dringlichkeitsstufe auf korrelierte Weise nach Bestimmung, dass die Krankheit, die das Nutztier belastet, das andere Nutztier belastet, beinhaltet.

4. Benachrichtigungsverfahren nach Anspruch 3, wobei
das Identifizieren ein Identifizieren einer Adresse einer Kommunikationsvorrichtung eines Arbeiters, der das andere Nutztier züchtet, nach Bestimmung, dass die Krankheit, die das Nutztier belastet, das andere Nutztier belastet, beinhaltet, wobei die Adresse durch Bezugnehmen auf eine dritte Speichereinheit identifiziert wird, die eine Adresse einer Kommunikationsvorrichtung eines Arbeiters, der jedes Nutztier in der Nutztiergruppe züchtet, speichert, und
das Ausgeben ein Ausgeben von dem Krankheitsnamen der Krankheit, die das Nutztier belastet, der identifizierten Dringlichkeitsstufe und Identifikationsinformationen des anderen Nutztiers auf korrelierte Weise zu der identifizierten Adresse beinhaltet.

5. Benachrichtigungsprogramm, das von einem Computer ausgeführt wird, wobei das Benachrichtigungsprogramm bewirkt, dass der Computer einen Vorgang ausführt, umfassend:
Empfangen eines ersten Zeitpunkts, zu dem ein Ausbruch einer Krankheit bei einem Nutztier aus einer Nutztiergruppe bestätigt wird, und eines Krankheitsnamen der Krankheit, die das Nutztier belastet;
Identifizieren einer Krankheitsdauer, die dem empfangenen Krankheitsnamen der Krankheit, die das Nutztier belastet, entspricht, durch Bezugnehmen auf eine erste Speichereinheit (1002), die einen Krankheitsnamen einer Krankheit und eine Krankheitsdauer der Krankheit auf korrelierte Weise speichert;
Beziehen von Messergebnissen einer Schrittzahl eines anderen Nutztiers, das sich von dem Nutztier in der Nutztiergruppe unterscheidet, von einer zweiten Speichereinheit (1004), die jedes Datum für jeweilige Zeitfenster speichert, einem Messergebnis des anderen Nutztiers für ein erstes Zeitfenster, das den ersten Zeitpunkt beinhaltet, und einem Messergebnis des anderen Nutztiers für ein zweites Zeitfenster, das einen zweiten Zeitpunkt beinhaltet, der von dem ersten Zeitpunkt um mindestens einen Zeitraum der identifizierten Krankheitsdauer zurückverfolgt wurde;
Bestimmen, ob die Krankheit, die das Nutztier belastet, das andere Nutztier belastet, auf der Basis des bezogenen Messergebnisses des anderen Nutztiers für das erste Zeitfenster, des bezogenen Messergebnisses des anderen Nutztiers für das zweite Zeitfenster und eines Grenzwerts, der sich auf die Schrittzahl bezieht, die für die Krankheit spezifisch ist, die das Nutztier belastet; und
Ausgeben von einem Krankheitsnamen der Krankheit des Nutztiers und Identifikationsinformationen des anderen Nutztiers nach Bestimmung, dass die Krankheit, die das Nutztier belastet, das andere Nutztier belastet.

6. Benachrichtigungsvorrichtung (102), umfassend:
eine Empfangseinheit (1001), die einen ersten Zeitpunkt, zu dem ein Ausbruch einer Krankheit bei einem Nutztier aus einer Nutztiergruppe bestätigt wird, und einen Krankheitsnamen der Krankheit, die das Nutztier belastet, empfängt;
eine Identifizierungseinheit (1003), die eine Krankheitsdauer, die dem empfangenen Krankheitsnamen der Krankheit, die das Nutztier belastet, entspricht, durch Bezugnehmen auf eine erste Speichereinheit (1002), die einen Krankheitsnamen einer Krankheit und eine Krankheitsdauer der Krankheit auf korrelierte Weise speichert, identifiziert;
eine Bezugseinheit (1005), die Messergebnisse einer Schrittzahl eines anderen Nutztiers, das sich von dem Nutztier in der Nutztiergruppe unterscheidet, von einer zweiten Speichereinheit (1004), die jedes Datum für jeweilige Zeitfenster speichert, bezieht, ein Messergebnis des anderen Nutztiers für ein erstes Zeitfenster, das den ersten Zeitpunkt beinhaltet, und ein Messergebnis des anderen Nutztiers für ein zweites Zeitfenster, das einen zweiten Zeitpunkt beinhaltet, der von dem ersten Zeitpunkt um mindestens einen Zeitraum der von der Identifizierungseinheit (1003) identifizierten Krankheitsdauer zurückverfolgt wurde;
eine Bestimmungseinheit (1006), die auf der Basis des Messergebnisses des anderen Nutztiers für das erste Zeitfenster und des Messergebnisses des anderen Nutztiers für das zweite Zeitfenster, die durch das Beziehen bezogen wurden, und eines Grenzwerts, der sich auf die Schrittzahl bezieht, die für die Krankheit spezifisch ist, die das Nutztier belastet, bestimmt, ob die Krankheit, die das Nutztier belastet, das andere Nutztier belastet; und
eine Ausgabeeinheit (1007), die einen Krankheitsnamen der Krankheit des Nutztiers und Identifikationsinformationen des anderen Nutztiers nach Bestimmung, dass die Krankheit, die das Nutztier belastet, das andere Nutztier belastet, durch die Bestimmungseinheit (1006) ausgibt.

## Revendications

1. Procédé de notification exécuté par un ordinateur, le procédé de notification comprenant les étapes ci-dessous consistant à :
recevoir un premier instant auquel l'apparition d'une maladie est confirmée chez un animal d'élevage parmi un groupe d'animaux d'élevage, et un nom de maladie de la maladie dont souffre l'animal d'élevage ;
identifier une durée de maladie correspondant au nom de maladie reçu de la maladie dont souffre l'animal d'élevage, en faisant référence à une première unité de stockage (1002) qui stocke un nom de maladie d'une maladie et une durée de maladie de la maladie, d'une manière corrélée ;
acquérir, auprès d'une deuxième unité de stockage (1004) qui stocke, pour des intervalles de temps respectifs de chaque date, des résultats de mesure d'un comptage de pas d'un autre animal d'élevage, différent de l'animal d'élevage du groupe d'animaux d'élevage, un résultat de mesure de l'autre animal d'élevage pour un premier intervalle de temps qui inclut le premier instant, et un résultat de mesure de l'autre animal d'élevage pour un second intervalle de temps qui inclut un second instant retracé à partir du premier instant, au moins d'une période de la durée de maladie identifiée ;
déterminer si la maladie dont souffre l'animal d'élevage affecte l'autre animal d'élevage, sur la base du résultat de mesure acquis de l'autre animal d'élevage pour le premier intervalle de temps, du résultat de mesure acquis de l'autre animal d'élevage pour le second intervalle de temps, et d'une valeur de seuil connexe au comptage de pas spécifique à la maladie dont souffre l'animal d'élevage ; et
fournir en sortie un nom de maladie de la maladie de l'animal d'élevage et des informations d'identification de l'autre animal d'élevage, lorsqu'il est déterminé que la maladie dont souffre l'animal d'élevage affecte l'autre animal d'élevage.

2. Procédé de notification selon la revendication 1, dans lequel :
la deuxième unité de stockage (1004) stocke en outre des résultats de mesure du comptage de pas de l'animal d'élevage pour les intervalles de temps respectifs ;
l'étape d'acquisition consiste à acquérir, auprès de la deuxième unité de stockage (1004), un résultat de mesure du comptage de pas de l'animal d'élevage pour le premier intervalle de temps et un résultat de mesure du comptage de pas de l'autre animal d'élevage pour le second intervalle de temps ;
le procédé de notification comprenant en outre l'étape ci-dessous consistant à :
calculer la valeur de seuil sur la base du résultat de mesure acquis du comptage de pas de l'animal d'élevage pour le premier intervalle de temps et du résultat de mesure acquis du comptage de pas de l'autre animal d'élevage pour le second intervalle de temps ; et
l'étape de détermination consiste à déterminer si une maladie dont souffre l'animal d'élevage affecte l'autre animal d'élevage, sur la base du résultat de mesure de l'autre animal d'élevage pour le premier intervalle de temps, du résultat de mesure de l'autre animal d'élevage pour le second intervalle de temps, et de la valeur de seuil calculée.

3. Procédé de notification selon la revendication 1 ou 2, dans lequel :
la première unité de stockage (1002) stocke un nom de maladie d'une maladie, une durée de maladie de la maladie, et un niveau d'urgence de la maladie, de manière corrélée ;
l'étape d'identification consiste à identifier une durée de maladie et un niveau d'urgence qui correspondent au nom de maladie reçu de la maladie affectant l'animal d'élevage, en faisant référence à la première unité de stockage (1002) ; et
l'étape de fourniture en sortie consiste à fournir en sortie le niveau d'urgence identifié, d'une manière corrélée, lorsqu'il est déterminé que la maladie dont souffre l'animal d'élevage affecte l'autre animal d'élevage.

4. Procédé de notification selon la revendication 3, dans lequel :
l'étape d'identification consiste à identifier une adresse d'un dispositif de communication d'un travailleur élevant l'autre animal d'élevage, lorsqu'il est déterminé que la maladie dont souffre l'animal d'élevage affecte l'autre animal d'élevage, l'adresse étant identifiée en faisant référence à une troisième unité de stockage qui stocke une adresse d'un dispositif de communication d'un travailleur élevant chaque animal d'élevage du groupe d'animaux d'élevage ; et
l'étape de fourniture en sortie consiste à fournir en sortie, d'une manière corrélée, à l'adresse identifiée, le nom de maladie de la maladie dont souffre l'animal d'élevage, le niveau d'urgence identifié, et des informations d'identification de l'autre animal d'élevage.

5. Programme de notification exécuté par un ordinateur, le programme de notification amenant l'ordinateur à exécuter un processus comprenant les étapes ci-dessous consistant à :
recevoir un premier instant auquel l'apparition d'une maladie est confirmée chez un animal d'élevage parmi un groupe d'animaux d'élevage, et un nom de maladie de la maladie dont souffre l'animal d'élevage ;
identifier une durée de maladie correspondant au nom de maladie reçu de la maladie dont souffre l'animal d'élevage, en faisant référence à une première unité de stockage (1002) qui stocke un nom de maladie d'une maladie et une durée de maladie de la maladie, d'une manière corrélée ;
acquérir, auprès d'une deuxième unité de stockage (1004) qui stocke, pour des intervalles de temps respectifs de chaque date, des résultats de mesure d'un comptage de pas d'un autre animal d'élevage, différent de l'animal d'élevage du groupe d'animaux d'élevage, un résultat de mesure de l'autre animal d'élevage pour un premier intervalle de temps qui inclut le premier instant, et un résultat de mesure de l'autre animal d'élevage pour un second intervalle de temps qui inclut un second instant retracé à partir du premier instant, au moins d'une période de la durée de maladie identifiée ;
déterminer si la maladie dont souffre l'animal d'élevage affecte l'autre animal d'élevage, sur la base du résultat de mesure acquis de l'autre animal d'élevage pour le premier intervalle de temps, du résultat de mesure acquis de l'autre animal d'élevage pour le second intervalle de temps, et d'une valeur de seuil connexe au comptage de pas spécifique à la maladie dont souffre l'animal d'élevage ; et
fournir en sortie un nom de maladie de la maladie de l'animal d'élevage et des informations d'identification de l'autre animal d'élevage, lorsqu'il est déterminé que la maladie dont souffre l'animal d'élevage affecte l'autre animal d'élevage.

6. Appareil de notification (102) comprenant :
une unité de réception (1001) qui reçoit un premier instant auquel l'apparition d'une maladie est confirmée chez un animal d'élevage parmi un groupe d'animaux d'élevage, et un nom de maladie de la maladie dont souffre l'animal d'élevage ;
une unité d'identification (1003) qui identifie une durée de maladie correspondant au nom de maladie reçu de la maladie dont souffre l'animal d'élevage, en faisant référence à une première unité de stockage (1002) qui stocke un nom de maladie d'une maladie et une durée de maladie de la maladie, d'une manière corrélée ;
une unité d'acquisition (1005) qui acquiert, auprès d'une deuxième unité de stockage (1004) qui stocke, pour des intervalles de temps respectifs de chaque date, des résultats de mesure d'un comptage de pas d'un autre animal d'élevage, différent de l'animal d'élevage du groupe d'animaux d'élevage, un résultat de mesure de l'autre animal d'élevage pour un premier intervalle de temps qui inclut le premier instant, et un résultat de mesure de l'autre animal d'élevage pour un second intervalle de temps qui inclut un second instant retracé à partir du premier instant, au moins d'une période de la durée de maladie identifiée par l'unité d'identification (1003) ;
une unité de détermination (1006) qui détermine si la maladie dont souffre l'animal d'élevage affecte l'autre animal d'élevage, sur la base du résultat de mesure de l'autre animal d'élevage pour le premier intervalle de temps, du résultat de mesure de l'autre animal d'élevage pour le second intervalle de temps, acquis au cours de l'étape d'acquisition, et d'une valeur de seuil connexe au comptage de pas spécifique à la maladie dont souffre l'animal d'élevage ; et
une unité de fourniture en sortie (1007) qui fournit en sortie un nom de maladie de la maladie de l'animal d'élevage et des informations d'identification de l'autre animal d'élevage, lorsqu'il est déterminé, par l'unité de détermination (1006), que la maladie dont souffre l'animal d'élevage affecte l'autre animal d'élevage.
